# EUROPEAN PATENT SPECIFICATION

(11) **EP 2 300 440 B1**
(45) Date of publication and mention of the grant of the patent: **27.02.2013**
(21) Application number: 09757461.0
(22) Date of filing: 29.05.2009
(51) Int. Cl.: C07D 239/22, C07D 401/04, C07D 471/04, C07D 405/04, A61K 31/517, A61K 31/519, A61P 29/00

(54) **3,4-DIHYDROPYRIMIDINE TRPA1 ANTAGONISTS**
3,4-DIHYDROPYRIMIDIN-TRPA1-ANTAGONISTEN
ANTAGONISTES TRPA1 3,4-DIHYDROPYRIMIDINE

(30) Priority: 02.06.2008 EP 08157398
(43) Date of publication of application: 30.03.2011
(73) Proprietor: Janssen Pharmaceutica, N.V., 2340 Beerse (BE)
(72) Inventor: GIJSEN, Henricus Jacobus Maria, B-2340 Beerse (BE); BERTHELOT, Didier, Jean-Claude, B-2340 Beerse (BE); DE CLEYN, Michel, Anna, Jozef, B-2340 Beerse (BE)
(74) Representative: Verberckmoes, Filip Gerard
(86) International application number: PCT/EP2009/056593
(87) International publication number: WO 2009/147079

(56) References cited:
- WO-A-2007/073505

## Description

The present invention is related to novel 3,4-dihydropyrimidine compounds of formula (I) having TRPA1 receptor antagonistic properties, pharmaceutical compositions comprising these compounds, chemical processes for preparing these compounds and their use in the treatment of diseases linked to the modulation of the TRPA1 receptors in animals, in particular humans.

The Transient Receptor Potential A1 (TRPA1), formerly named ANKTM1, receptor belongs to the transient receptor potential (TRP) family of cation-selective channels which have been shown to transduce mechanical, thermal, and pain-related inflammatory signals (see e.g. Biochimica et Biophysica Acta 1772 (2007) 989-1003; Cell 124 (2006) 1123-1125).

TRPA1 is a non-selective calcium permeable channel, modulating membrane potential by modulating the flux of cations such as calcium and sodium ions. The miss-regulation of ion channels is often associated with pathological conditions, and compounds which are able to modulate one or more functions of ion-channels, including TRPA1, are of great interest as possible therapeutic agents. Activators or agonists of the TRPA1 receptor, such as isothiocyanates (allylisothiocyanate, the pungent component of mustard) and tear gasses, cause acute pain and neurogenic inflammation. (see e.g. PNAS 103 (2007) 13519-13524; Cell 124(2006) 1269-1282). Modulation of the TRPA1 receptor may lead to an improved homeostasis of ion-flux and membrane potential.

In a formalin-induced pain model, TRPA1 has been shown to be the principal site of formalin's pain-producing action *in vivo*, and activation of TRPA1 underlies the physiological and behavioral responses associated with this model of pain hypersensitivity. Formalin induced activation of the TRPA1 channel can be attenuated by TRPA1 antagonists (PNAS 103 (2007) 13525-13530.)

There is an interest in the identification and development of ligands to the TRPA1 receptor to be of possible use in the prevention, treatment, or alleviating symptoms of a disease or condition associated with TRPA1 (see for example WO-2007/073505 or WO-2007/098252 for patents claiming TRPA1 antagonists).

3,4-Dihydropyrimidines without annulation on the dihydropyrimidine 5,6-double bond, as exemplified by formula (II) have been claimed as TRPA1 antagonists in WO-2007/073505, but in our hands such compounds showed no or very weak antagonism to the human TRPA1 receptor. A few dihydropyrimidinones have been described in the literature (for example compound A: Indian J, Chem., Section B, 43B (2004), 135-140. Compounds B and C: Phosphorous and Sulfur 39, (1988), 211-16, also see Latvijas PSR Zinatnu Akademijas Vestis, Kimijas Serija (1968), 324-7). Dihydropyrimidone compounds of formula D have been claimed as eye lens clarification agents in EP 489991.

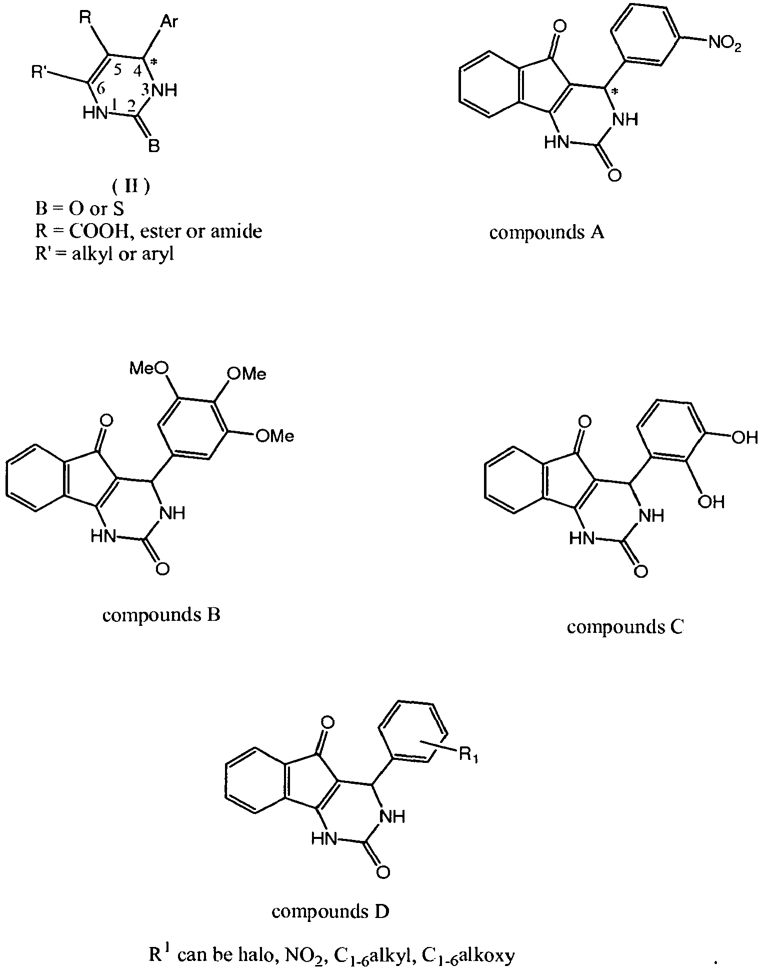

The present invention relates to novel compounds of formula (I) 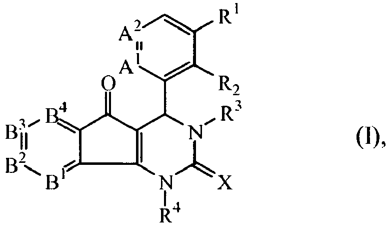 including any stereochemically isomeric form thereof wherein
A¹ and A² are both CR⁵, or one of A¹ or A² is N and the other is CR⁵, wherein each R⁵ is independently selected from hydrogen, halo, C₁₋₆alkyl, C₁₋₆alkyloxy, polyhaloC₁₋₆alkyl or polyhaloC₁₋₆alkyloxy;
B¹, B², B³ and B⁴ are all CH, or one of B¹, B² B³ and B⁴ is N and the other are CH;
X is O or S;
R¹ is halo, hydroxy, cyano, amino, C₁₋₆alkyl, hydroxyC₁₋₆alkyl, C₁₋₆alkyloxy, polyhaloC₁₋₆alkyloxy, aryl, aryloxy, or OR⁶ wherein R⁶ is C₁₋₆alkyl substituted with hydroxy, C₁₋₄alkyloxy, aryl, aryloxy, C₁₋₄alkylcarbonyl, C₁₋₄alkylcarbonyloxy, or NR⁷R⁸ wherein R⁷ and R⁸ are each independently selected from hydrogen or C₁₋₄alkyl;
R² is hydrogen, fluoro, C₁₋₄alkyl or C₁₋₄alkyloxy; and R¹ and R² may be taken together to form a -O-CH₂-O- or -O-CH₂-CH₂-O- radical;
R³ is hydrogen, C₁₋₄alkyl, or arylC₁₋₂alkyl;
R⁴ is hydrogen, C₁₋₄alkyl, or arylC₁₋₂alkyl;
each aryl is independently from the other selected from a phenyl substituted with 1, 2 or 3 substituents each independently selected from hydrogen, halo, hydroxy, C₁₋₄alkyl, polyhaloC₁₋₄alkyl, C₁₋₄alkyloxy, polyhaloC₁₋₄alkyloxy, cyano, nitro, amino, or mono- or di-(C₁₋₄alkyl)amino;
provided that when X is O, and A¹ and A² are both CR⁵ wherein R⁵ is hydrogen, and R² is hydrogen or C₁₋₄alkyloxy, and R³ and R⁴ are hydrogen, than R¹ is not halo, C₁₋₆alkyl or C₁₋₆alkyloxy;
or a pharmaceutically acceptable acid addition salt thereof, or a solvate thereof, or an
N-oxide thereof.

The proviso is intended to disclaim the compounds provided by EP-0,489,991 or the CAS numbers 371142-26-6, 371131-70-3, 371129-06-5 and 371120-50-2.

As used in the foregoing definitions :
- halo is generic to fluoro, chloro, bromo and iodo;
- C₁₋₄alkyl defines straight and branched chain saturated hydrocarbon radicals having from 1 to 4 carbon atoms such as, for example, methyl, ethyl, propyl, butyl, 1-methylethyl, 2-methylpropyl and the like;
- C₁₋₆alkyl is meant to include C₁₋₄alkyl and the higher homologues thereof having 5-6 carbon atoms, such as, for example, 2-methylbutyl, pentyl, hexyl and the like;
- polyhaloC₁₋₄alkyl is defined as polyhalosubstituted C₁₋₄alkyl, in particular C₁₋₄alkyl (as hereinabove defined) substituted with 2 to 6 halogen atoms such as difluoromethyl, trifluoromethyl, trifluoroethyl, and the like;
- polyhaloC₁₋₆alkyl is defined as polyhalosubstituted C₁₋₆alkyl, in particular C₁₋₆alkyl (as hereinabove defined) substituted with 2 to 6 halogen atoms such as difluoromethyl, trifluoromethyl, trifluoroethyl, and the like.

The term "stereochemically isomeric forms" as used hereinbefore defines all the possible isomeric forms which the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Stereochemically isomeric forms of the compounds of formula (I) are obviously intended to be embraced within the scope of this invention.

All compounds of formula (I) have at least one chiral carbon atom as indicated in the figure below with the *. Both the R and S configurations of said chiral carbon atom are intended to be covered under the term "stereochemically isomeric forms". Compounds of formula (I) that have been prepared as a mixture of the R and S enantiomers can be resolved into their individual enantiomers using art-known resolution techniques such as e.g. Supercritical Fluid Chromatography (SFC).

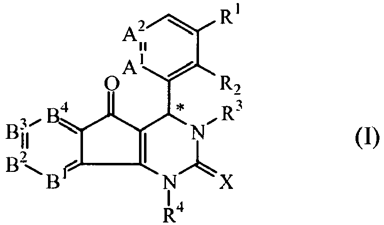

The absolute stereochemical configuration of the compounds of formula (I) and of the intermediates used in their preparation may easily be determined by those skilled in the art while using well-known methods such as, for example, X-ray diffraction or VCD.

Furthermore, some compounds of formula (I) and some of the intermediates used in their preparation may exhibit polymorphism. It is to be understood that the present invention encompasses any polymorphic forms possessing properties useful in the treatment of the conditions noted hereinabove.

The pharmaceutically acceptable acid addition salts as mentioned hereinabove are meant to comprise the therapeutically active non-toxic acid addition salt forms that the compounds of formula (I) are able to form. These pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (*i.e*. butanedioic acid), maleic, fumaric, malic, tartaric, citric, methanesulfonic, ethanesulfonic, benzenesulfonic, p-toluenesulfonic, cyclamic, salicylic, *p*-aminosalicylic, pamoic and the like acids.

Conversely said salt forms can be converted by treatment with an appropriate base into the free base form.

The compounds of formula (I) may exist in both unsolvated and solvated forms. The term 'solvate' is used herein to describe a molecular association comprising a compound of the invention and one or more pharmaceutically acceptable solvent molecules, e.g. water or ethanol. The term 'hydrate' is used when said solvent is water.

Interesting compounds of formula (I) are those compounds of formula (I) wherein one or more of the following restrictions apply :
a) A¹ and A² are both CR⁵ wherein each R⁵ is independently selected from hydrogen or halo; or
b) one of A¹ and A² is CR⁵ wherein R⁵ is hydrogen or halo and the other of A¹ and A² is N; or
c) B¹, B², B³ and B⁴ are all CH; or
d) R² is hydrogen or C₁₋₄alkyloxy; or
e) R¹ and R² may be taken together to form a -O-CH₂-O-; or
f) R³ is hydrogen or methyl; or
g) R⁴ is hydrogen, methyl or phenylmethyl;
h) aryl is phenyl, or phenyl substituted with halo, trifluoromethyl, cyano, or C₁₋₄alkyloxy; or
i) the stereogenic center at C* has the R-configuration.

A particular group of compounds of formula (I) are those compounds of formula (I) wherein X is S.

Another particular group of compounds of formula (I) are those compounds of formula (I) wherein A¹ and A2 are both CR⁵ wherein each R⁵ is independently selected from hydrogen or halo; R¹ is halo, hydroxy, C₁₋₆alkyloxy, or C₁₋₆alkyl wherein C₁₋₆alkyl can be optionally substituted with C₁₋₄alkoxy, hydroxy, C₁₋₄alkylcarbonyloxy, or NR⁷R⁸ wherein R⁷ and R⁸ are C₁₋₄alkyl.

Yet another particular group of compounds of formula (I) are those compounds of formula (I) wherein A¹ and A² are both CR⁵ wherein each R⁵ is independently selected from hydrogen; B¹, B², B³ and B⁴ are all CH; X is S; R¹ is C₁₋₄alkyloxy wherein C₁₋₄alkyl is optionally substituted with C₁₋₄alkoxy or hydroxy and the stereogenic center at C* has the R-configuration.

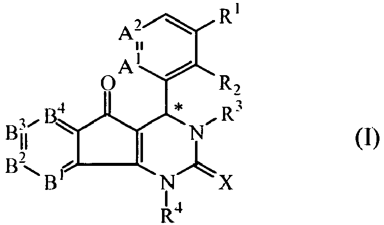

### General synthesis (Synthetic routes)

In general compounds of formula (I) can be synthesized via a multicomponent condensation reaction between a 1,3-dicarbonyl compound (III), an aldehyde (IV), and (thio)urea (V).

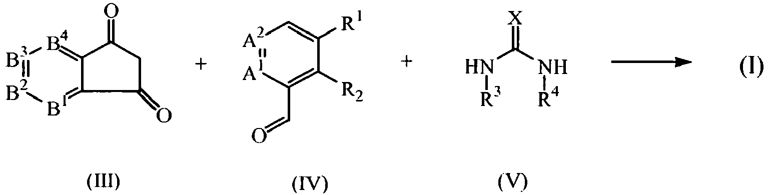

Compounds of formula (I-a), defined as compounds of formula (I) wherein R³ and R⁴ represent hydrogen, can be synthesized via a multicomponent condensation reaction between a 1,3-dicarbonyl compound (III), an aldehyde (IV), and (thio)urea (V), known as the Biginelli reaction. (For general Biginelli methodology see e.g. Tetrahedron 336 (1993) 6937-6963. For Biginelli reactions more specific for compounds of formula (I) see e.g. Indian J. Chem. 43B (2004), 135-140).

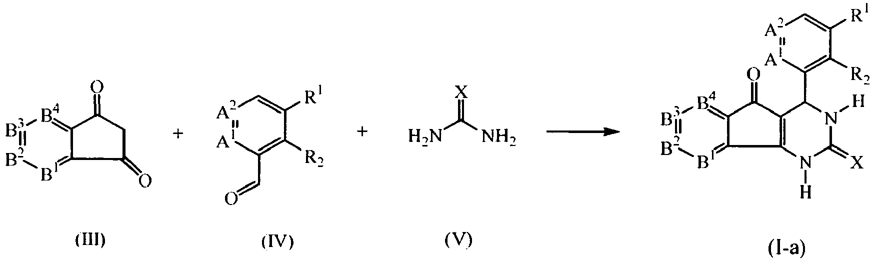

This reaction can be run by mixing and heating the components in the presence of a Lewis or mineral acidic catalyst and with or without the presence of a solvent. Examples of useful catalysts are HCl, HBr, H₂SO₄, acetic acid, trifluoric acid, p-toluenesulfonic acid, trimethylsilylchloride, trimethylsilyliodide, boron trifluoride etherate, copper (I) chloride, ferric chloride, indium(III) chloride, ytterbium triflate, cerium (III) chloride, zirconium (IV) chloride, zirconium (IV) oxychloride, lithiumbromide, phenylpyruvic acid, calcium chloride, polyphosphate ester, or solid clay acid catalysts such as montmorillonite KSF clay, or combinations of these catalysts.

Useful solvents are preferably polar solvents such as acetonitrile, acetic acid, methanol, ethanol, or other alcohols, tetrahydrofuran, dimethylformamide, dimethylacetamide, N-methylpyrrolidone, or dioxane.

Heating can be done under conventional thermal conditions or under microwave conditions.

Compounds of formula (I-a) can also be prepared in a two step procedure, where the Knoevenagel condensation product between (III) and (IV) is reacted with (V), as described in, e.g. Phosphorous and Sulfur 39, (1988), 211-16 or Chem. Heterocycl. Compd. (Engl.Transl.) 24 (1988), 936-941.

Compounds of formula (I-b) and (I-c), defined as compounds of formula (I) wherein X represents O and R³ and/or R⁴ are not hydrogen, can be prepared by reacting compounds of formula (I-a-1), defined as compounds of formula (I) wherein X represents O, and R³ and R⁴ represent hydrogen, with an intermediate (VI), wherein L is a leaving group such as halo, methanesulfonyloxy, benzenesulfonyloxy, trifluoromethanesulfonyloxy and the like reactive leaving groups, in the presence of a suitable base such as NaH or K₂CO₃ in a reaction-inert solvent such as e.g. THF, 1,4-dioxane, 2-propanone, or dimethylformamide.

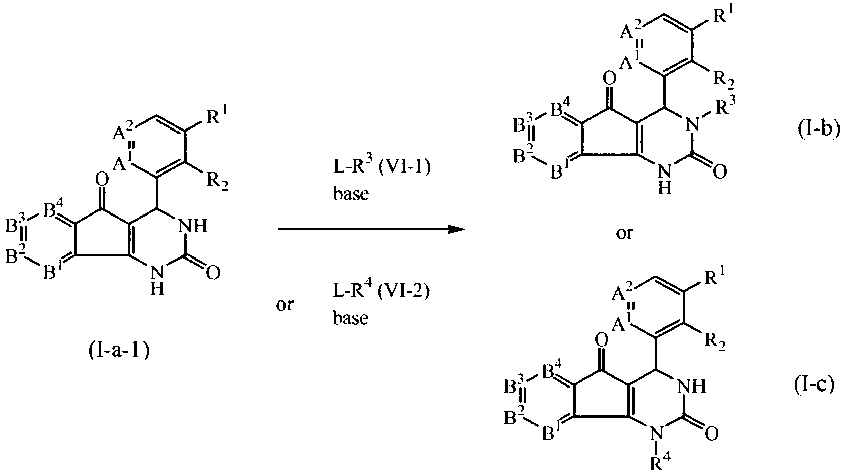

Compounds of formula (I-d) and (I-e), defined as compounds of formula (I) wherein X represents S and R³ and/or R⁴ are not hydrogen, can be prepared by alkylating compound (I-a-2), defined as compounds of formula (I) wherein X represents S, and R³ and R⁴ represent hydrogen, with reagent (VII), to give intermediate (VIII), which can be alkylated with an intermediate (VI-1) or (VI-2), wherein L is a leaving group such as halo, methanesulfonyloxy, benzenesulfonyloxy, trifluoromethanesulfonyloxy and the like reactive leaving groups, in the presence of a suitable base such as NaH or K₂CO₃ in a reaction-inert solvent such as e.g. 2-propanone, 1,4-dioxane, dimethylformamide, or THF to give intermediates (IX) and/or (X). Upon treatment of intermediates (IX) or (X) with TFA under thermal or microwave assisted heating compounds of formula (I-d) and (I-e) are obtained, respectively.

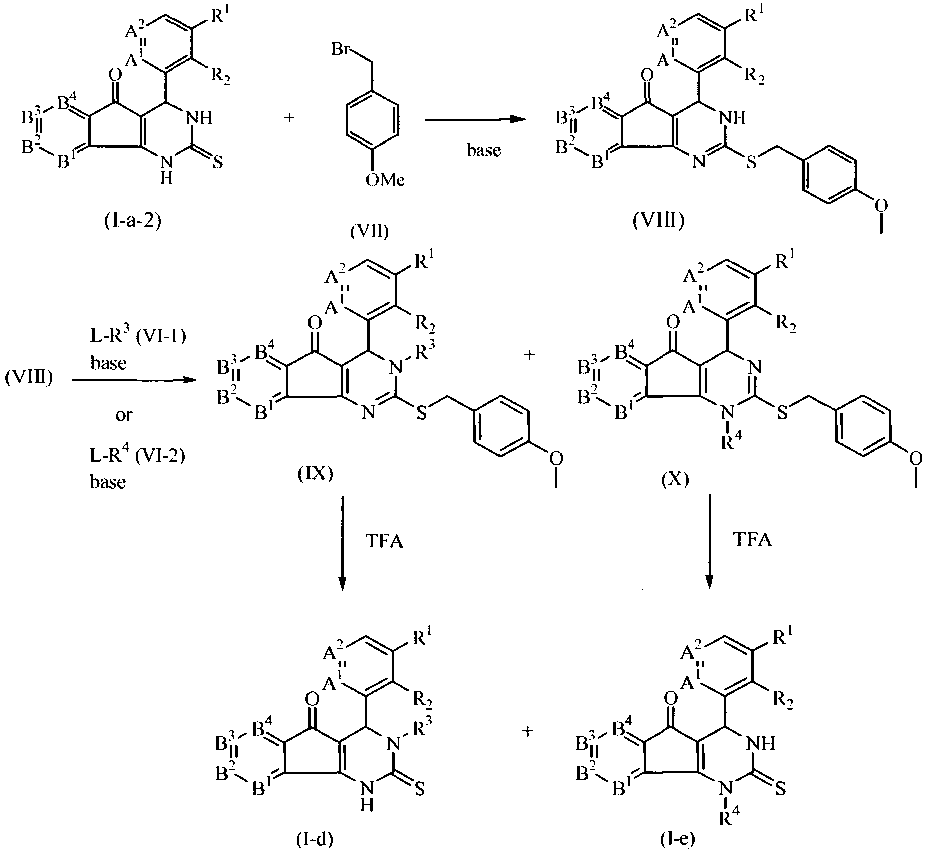

Substituents R¹ can be further derivatized via standard functional group transformations to prepare the desired final compounds of formula (I).

The starting materials and some of the intermediates are commercially available or may be prepared according to conventional reaction procedures generally known in the art.

The compounds of formula (I) as prepared in the hereinabove described processes may be synthesized in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I), including any stereochemically isomeric form thereof, or pharmaceutically acceptable acid addition salts, solvates or N-oxides thereof, possess transient receptor potential A1 receptor (TRPA1) antagonistic properties as demonstrated in the Pharmacological Examples. Pharmacological example D describes the methodology to measure TRPA1 antagonism and results are listed in Table 5.

Therefore the present compounds of formula (I) are useful as a medicine especially in the treatment of a condition or disease mediated by the TRPA1 receptor, in particular TRPA1 receptor antagonistic activity. Subsequently the present compounds may be used for the manufacture of a medicine for treatment of a condition or a disease mediated by TRPA1 activity, in particular TRPA1 antagonistic activity.

Preferably, the present invention also provides the use of a compound of formula (I) or a pharmaceutically acceptable salt thereof for the manufacture of a medicament for the treatment of conditions or diseases selected from TRPA1 mediated conditions or diseases.

Further, disclosed is a method of treatment of a condition mediated by TRPA1 activity, in a mammalian subject, which comprises administering to a mammal in need of such treatment a therapeutically effective amount of a compound of formula (I) or a pharmaceutically acceptable salt thereof.

TRPA1 mediated conditions or diseases are e.g. pain, chronic pain, touch sensitivity, itching sensitivity, skin irritation, post-surgical pain, cancer pain, neuropathic pain, inflammatory pain, migraine, diabetic neuropathy, psoriasis, eczema, dermatitis, post-herpetic neuralgia, urinary incontinence, pancreatitis, osteoarthritis, rheumatoid arthritis, oral mucositis, inhibition or stimulation of hair growth, lacrimation, ocular injuries, blepharospasm, and pulmonary irritation (e.g. cough), and stimulation of wound healing.

The term "treating" and "treatment', as used herein, refers to curative, palliative and prophylactic treatment, including reversing, alleviating, inhibiting the progress of, or preventing the disease, disorder or condition to which such term applies, or one or more symptoms of such disease, disorder or condition.

Additionally the present invention provides pharmaceutical compositions comprising at least one pharmaceutically acceptable carrier and a therapeutically effective amount of a compound of formula (I).

In order to prepare the pharmaceutical compositions of this invention, an effective amount of the particular compound, in base or acid addition salt form, as the active ingredient is combined in intimate admixture with at least one pharmaceutically acceptable carrier, which carrier may take a wide variety of forms depending on the form of preparation desired for administration. These pharmaceutical compositions are desirably in unitary dosage form suitable, preferably, for oral administration, rectal administration, percutaneous administration, parenteral injection, or topical administration.

For example in preparing the compositions in oral dosage form, any of the usual liquid pharmaceutical carriers may be employed, such as for instance water, glycols, oils, alcohols and the like in the case of oral liquid preparations such as suspensions, syrups, elixirs and solutions; or solid pharmaceutical carriers such as starches, sugars, kaolin, lubricants, binders, disintegrating agents and the like in the case of powders, pills, capsules and tablets. Because of their easy administration, tablets and capsules represent the most advantageous oral dosage unit form, in which case solid pharmaceutical carriers are obviously employed. For parenteral injection compositions, the pharmaceutical carrier will mainly comprise sterile water, although other ingredients may be included in order to improve solubility of the active ingredient. Injectable solutions may be prepared for instance by using a pharmaceutical carrier comprising a saline solution, a glucose solution or a mixture of both. Injectable suspensions may also be prepared by using appropriate liquid carriers, suspending agents and the like. In compositions suitable for percutaneous administration, the pharmaceutical carrier may optionally comprise a penetration enhancing agent and/or a suitable wetting agent, optionally combined with minor proportions of suitable additives which do not cause a significant deleterious effect to the skin. Said additives may be selected in order to facilitate administration of the active ingredient to the skin and/or be helpful for preparing the desired compositions. These topical compositions may be administered in various ways, e.g., as a transdermal patch, a spot-on or an ointment. Addition salts of the compounds of formula (I), due to their increased water solubility over the corresponding base form, are obviously more suitable in the preparation of aqueous compositions.

It is especially advantageous to formulate the pharmaceutical compositions of the invention in dosage unit form for ease of administration and uniformity of dosage. "Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions of the present invention may take the form of solid dose forms, for example, tablets (both swallowable and chewable forms), capsules or gelcaps, prepared by conventional means with pharmaceutically acceptable excipients and carriers such as binding agents (e.g. pregelatinised maize starch, polyvinylpyrrolidone, hydroxypropylmethylcellulose and the like), fillers (e.g. lactose, microcrystalline cellulose, calcium phosphate and the like), lubricants (e.g. magnesium stearate, talc, silica and the like), disintegrating agents (e.g. potato starch, sodium starch glycollate and the like), wetting agents (e.g. sodium laurylsulphate) and the like. Such tablets may also be coated by methods well known in the art.

Liquid preparations for oral administration may take the form of e.g. solutions, syrups or suspensions, or they may be formulated as a dry product for admixture with water and/or another suitable liquid carrier before use. Such liquid preparations may be prepared by conventional means, optionally with other pharmaceutically acceptable additives such as suspending agents (e.g. sorbitol syrup, methylcellulose, hydroxypropylmethylcellulose or hydrogenated edible fats), emulsifying agents (e.g. lecithin or acacia), non-aqueous carriers (e.g. almond oil, oily esters or ethyl alcohol), sweeteners, flavours, masking agents and preservatives (e.g. methyl or propyl p-hydroxybenzoates or sorbic acid).

Pharmaceutically acceptable sweeteners useful in the pharmaceutical compositions of the invention comprise preferably at least one intense sweetener such as aspartame, acesulfame potassium, sodium cyclamate, alitame, a dihydrochalcone sweetener, monellin, stevioside sucralose (4,1',6'-trichloro-4,1',6'-trideoxygalactosucrose) or, preferably, saccharin, sodium or calcium saccharin, and optionally at least one bulk sweetener such as sorbitol, mannitol, fructose, sucrose, maltose, isomalt, glucose, hydrogenated glucose syrup, xylitol, caramel or honey. Intense sweeteners are conveniently used in low concentrations. For example, in the case of sodium saccharin, the said concentration may range from about 0.04% to 0.1% (weight/volume) of the final formulation. The bulk sweetener can effectively be used in larger concentrations ranging from about 10% to about 35%, preferably from about 10% to 15% (weight/volume).

The pharmaceutically acceptable flavours which can mask the bitter tasting ingredients in the low-dosage formulations are preferably fruit flavours such as cherry, raspberry, black currant or strawberry flavour. A combination of two flavours may yield very good results. In the high-dosage formulations, stronger pharmaceutically acceptable flavours may be required such as Caramel Chocolate, Mint Cool, Fantasy and the like. Each flavour may be present in the final composition in a concentration ranging from about 0.05% to 1% (weight/volume). Combinations of said strong flavours are advantageously used. Preferably a flavour is used that does not undergo any change or loss of taste and/or color under the circumstances of the formulation.

The compounds of formula (I) may be formulated for parenteral administration by injection, conveniently intravenous, intra-muscular or subcutaneous injection, for example by bolus injection or continuous intravenous infusion. Formulations for injection may be presented in unit dosage form, e.g. in ampoules or multi-dose containers, including an added preservative. They may take such forms as suspensions, solutions or emulsions in oily or aqueous vehicles, and may contain formulating agents such as isotonizing, suspending, stabilizing and/or dispersing agents. Alternatively, the active ingredient may be present in powder form for mixing with a suitable vehicle, e.g. sterile pyrogen-free water, before use.

The compounds of formula (I) may also be formulated in rectal compositions such as suppositories or retention enemas, e.g. containing conventional suppository bases such as cocoa butter and/or other glycerides.

The compounds of formula (I) may also be formulated in ophthalmic compositions for topical application to the eye such as an isotonic, non-irritating, aqueous solution.

Those of skill in the treatment of diseases linked to the mediation of the TRPA1 receptor will easily determine the therapeutically effective amount of a compound of formula (I) from the test results presented hereinafter. In general it is contemplated that a therapeutically effective dose will be from about 0.001 mg/kg to about 50 mg/kg of body weight, more preferably from about 0.01 mg/kg to about 10 mg/kg of body weight of the patient to be treated. It may be appropriate to administer the therapeutically effective dose in the form of two or more sub-doses at appropriate intervals throughout the day. Said sub-doses may be formulated as unit dosage forms, for example each containing from about 0.1 mg to about 1000 mg, more particularly from about 1 to about 500 mg, of the active ingredient per unit dosage form.

As used herein, a "therapeutically effective amount" of a compound, is the quantity of a compound which, when administered to an individual or animal, results in a sufficiently high level of that compound in the individual or animal to cause a discernible increase or decrease in stimulation of TRPA1 receptors.

The exact dosage and frequency of administration depends on the particular compound of formula (I) used, the particular condition being treated, the severity of the condition being treated, the age, weight and general physical condition of the particular patient as well as the other medication, the patient may be taking, as is well known to those skilled in the art. Furthermore, said "therapeutically effective amount" may be lowered or increased depending on the response of the treated patient and/or depending on the evaluation of the physician prescribing the compounds of the instant invention. The effective daily amount ranges mentioned hereinabove are therefore only guidelines.

### Experimental part

"DIPE" is defined as diisopropyl ether, "DMF" is defined as *N,N*-dimethylformamide, "DMSO" is defined as dimethyl sulfoxide, "DCM" or "CH₂Cl₂" is defined as dichloromethane and "THF" is defined as tetrahydrofuran. The term 'CH₃CN' means acetonitrile, 'K₂CO₃' means potassium carbonate, 'CH₃OH' means methanol, 'MgSO₄' means magnesium sulphate, 'NaH' means sodium hydride, 'EtOH' means ethanol, 'TFA' means trifluoroacetic acid, 'EtOAc' means ethyl acetate.

### High-Performance Liquid Chromatography purification methods :

### - purification methods A

The product was purified by reversed phase high-performance liquid chromatography (Shandon Hyperprep^{®} C18 BDS (Base Deactivated Silica) 8 µm, 250 g, I.D. 5 cm). A gradient with three mobile phases was applied (phase A: a 0.25 % NH₄HCO₃ solution in water; phase B: CH₃OH; phase C: CH₃CN). The desired fractions were collected and worked-up.

### - purification method B

The product was purified by reversed phase high-performance liquid chromatography (Shandon Hyperprep® C18 BDS (Base Deactivated Silica) 8 µm, 250 g, I.D. 5 cm). A gradient with three mobile phases was applied (phase A: 90 % of a 0.5 % NH₄OAc solution in water+ 10 % CH₃CN; phase B: CH₃OH; phase C: CH₃CN). The desired fractions were collected and worked-up.

### A. Synthesis of the intermediates

### Example A.1

| | | |
|---|---|---|
| Preparation of | 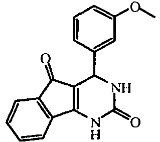 | intermediate (1) |

A mixture of urea (8 mmol), indene-1,3(2H)-dione (7 mmol), 3-methoxybenzaldehyde (4 mmol) and concentrated aqueous HCl (20 drops) in acetonitrile (30 ml) was heated in a microwave oven at 110°C for 20 minutes. The product was filtered off and dried overnight under vacuum. The product was suspended in acetonitrile and refluxed for I hour. The solid product was filtered off and dried under vacuum overnight, yielding intermediate (1) used in the next reaction step without further purification.

### Example A.2

| | | |
|---|---|---|
| Preparation of | 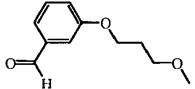 | intermediate (2) |

A mixture of 3-hydroxybenzaldehyde (10 mmol), 3-methoxy-1-bromopropane (12 mmol) and K₂CO₃ (10 mmol) in methanol (3 ml) was stirred and heated in a microwave oven at 100°C for 15 minutes. The obtained mixture was heated another 25 minutes at 110°C. The solvent was removed under a stream of nitrogen, and the solid residue was extracted with hexane (3 x 50 ml). The organic phase was concentrated, yielding 1.44 g of intermediate (2).

### Example A.3

| | | |
|---|---|---|
| Preparation of | 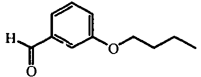 | intermediate (3) |

A mixture of 3-hydroxybenzaldehyde (2.88 g), 1-bromobutane (3 g) and K₂CO₃ (2.8 g) in methanol (6 ml) was reacted in a microwave oven for 30 minutes at 110°C. The solvent was evaporated under vacuum. The resultant solid precipitate was treated with heptane (50 ml) and the supernatant was decanted off (3 times), yielding intermediate (3) used as such in the next step without further analysis.

### Example A.4

| | | |
|---|---|---|
| Preparation of | 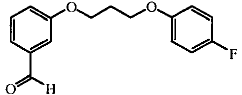 | intermediate (4) |

A mixture of 3-hydroxybenzaldehyde (21.5 mmol), 1-(3-bromopropoxy)-4-fluorobenzene (21.5 mmol) and K₂CO₃ (23 mmol) in methanol (7 ml) was stirred and heated in the microwave at 100°C for 60 minutes. The mother liquor was concentrated under reduced pressure, and then separated between water and DCM. The organic layer was dried (MgSO₄), concentrated and the residue was purified on silica gel column (eluent CH₂Cl₂/CH₃OH 100/0 to 99/1). The product fractions were collected and the solvent was evaporated, yielding 2.65 g of intermediate (4).

### Example A.5

| | | |
|---|---|---|
| Preparation of | 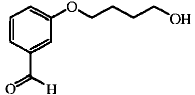 | intermediate (5) |

A mixture of 3-hydroxybenzaldehyde (25 mmol), 4-bromo-1-butanol 1-acetate (30 mmol) and K₂CO₃ (30 mmol) in methanol (8 ml) was stirred and heated in the microwave at 100°C for 60 minutes. The mother liquor was concentrated under reduced pressure, and then separated between water and DCM. The organic layer was dried (MgSO₄), concentrated and the residue was purified on silica gel column (eluent CH₂Cl/CH₃OH 100/0 to 97/3). The desired product fraction was collected and the solvent was evaporated, yielding 1.65 g of intermediate (5).

### Example A.6

| | | |
|---|---|---|
| a) Preparation of | 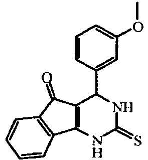 | intermediate (6) |

A mixture of indene-1,3(2H)-dione (7.5 mmol), 3-methoxybenzaldehyde (5 mmol), thiourea (5 mmol) and concentrated HCl (15 drops) in acetonitrile (20 ml) was heated in a microwave at 110°C for 30 minutes. The reaction mixture was filtered off hot. The solid filter residue was stirred in boiling acetonitrile, then filtered off and dried under vacuum at 50°C. The reaction was repeated 5 times, yielding 3.3 g of intermediate (6).

| | | |
|---|---|---|
| b) Preparation of | 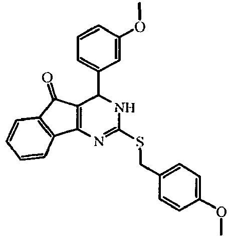 | intermediate (7) |

A solution of intermediate (6) (1.551 mmol) in DMF (5 ml) was stirred under a nitrogen atmosphere, then NaH (1.6 mmol) was added and the resulting reaction mixture was stirred at 50°C for 30 minutes, then cooled. 1-(Bromomethyl)-4-methoxybenzene (1.6 mmol) was added. The reaction mixture was stirred at room temperature for one hour. The solvent was evaporated. The residue was partitioned between CH₂Cl₂ and H₂O. The organic layer was separated, dried (MgSO₄), filtered and the solvent was evaporated. The residue was purified by column chromatography over silica gel (eluent: CH₂Cl₂/CH₃OH 99/1). The product fractions were collected and the solvent was evaporated. The residue was dried under vacuum at 50°C, yielding 170 mg of intermediate (7).

| | | |
|---|---|---|
| c) Preparation of | 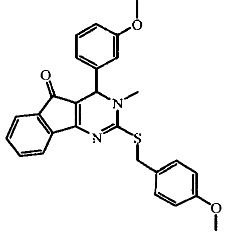 | intermediate (8) |
| and | 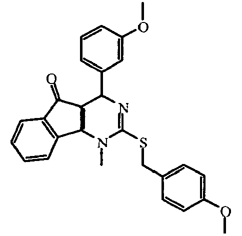 | intermediate (9) |

Reaction under nitrogen atmosphere. A mixture of intermediate (7) (4.52 mmol), iodomethane (5 mmol) and K₂CO₃ (10 mmol) in DMF (25 ml) was stirred at 80°C for one hour. The mixture was cooled and the solvent was evaporated. The residue was partitioned between DCM and water. The organic layer was separated, dried (MgSO₄), filtered and the solvent was evaporated. The residue was purified by column chromatography over silica gel (eluent : CH₂Cl₂). Two product fractions were collected and their solvent was evaporated, yielding 1 g of intermediate (8) and 0.1 g of intermediate (9).

### B. Preparation of the compounds

### Example B.1

| | | |
|---|---|---|
| Preparation of | 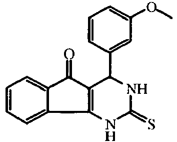 | compound (1) |

A mixture of 3-methoxybenzaldehyde (5 mmol), indene-1,3(2H)-dione (7.5 mmol) and thiourea (5 mmol) in acetonitrile (20 ml) was stirred at room temperature. Concentrated HCl (10 drops) was added and the reaction mixture was heated in a microwave oven at 110°C for 22 minutes. The reaction mixture was filtered, and the precipitate was washed with acetonitrile and dried in vacuo, yielding 0.62 g of compound (1).

### Example B.2

| | | |
|---|---|---|
| Preparation of | 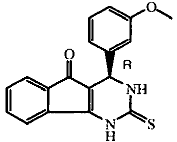 | compound (2) |
| and | 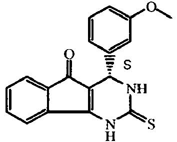 | compound (3) |

Compound (1) (1.5 g)was subjected to an enantiomer separation via Supercritical Fluid Chromatography (SFC, column: Diacel AS-H 20x250mm, mobile phase: 40% MeOH/60% CO₂ + 0.2% isopropylamine, isocratic at 40°C and 100 bar). Two product fraction groups were collected and their solvent was evaporated, yielding compound (2) and compound (3).

### Example B.3

| | | |
|---|---|---|
| Preparation of | 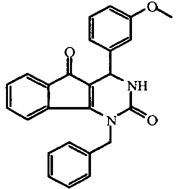 | compound (4) |

Intermediate (1) (0.8 mmol), (bromomethyl)benzene (1 mmol) and K₂CO₃ (1 mmol) were added to acetonitrile (40 ml). The reaction mixture was stirred overnight at room temperature and was then washed twice with water. The product mixture was first purified by flash column chromatography over silica gel (eluent: CH₂Cl₂/CH₃OH 90/10) and was then further purified by HPLC method A. The desired product fractions were collected and the solvent was evaporated. Methanol was added to the residue and the mixture was evaporated. The solid residue was dried under vacuum, yielding compound (4).

### Example B.4

| | | |
|---|---|---|
| Preparation of | 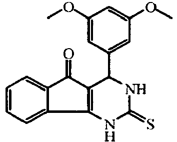 | compound (5) |

A mixture of 3,5-dimethoxybenzaldehyde (6.5 mmol), indene-1,3(2H)-dione (9.7 mmol) and thiourea (6.5 mmol) in acetonitrile (20 ml) was stirred at room temperature. Concentrated HCl (15 drops) was added and the reaction mixture was heated in a microwave oven at 110°C for 22 minutes. The reaction mixture was filtered, and the red precipitate was washed with acetonitrile and dried in vacuo. This fraction was triturated under methanol and some 2-propanone, filtered off and dried, yielding 0.6 g of compound (5).

### Example B.5

| | | |
|---|---|---|
| Preparation of | 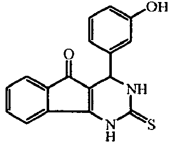 | compound (6) |

A mixture of 3-hydroxybenzaldehyde (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in a microwave oven at 110°C for 22 minutes. The reaction mixture was heated an additional 22 minutes at 110°C in the microwave oven. The reaction mixture was stirred at room temperature and DIPE (± 5ml) was slowly added dropwise. Stirring was continued at room temperature and after 10 minutes a red precipitate had formed. This was filtered off, washed with a mixture of CH₃CN/DIPE (3/1), followed by DIPE, then dried in vacuo. The obtained fraction was recrystallized from CH₃OH/CH₃CN, filtered off and dried, yielding 0.122 g of compound (6).

### Example B.6

| | | |
|---|---|---|
| Preparation of | 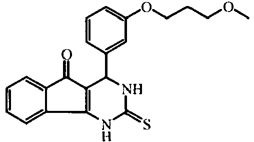 | compound (7) |

Intermediate (2) (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in the microwave oven at 110°C for 25 minutes. A precipitate was formed, which was filtered off and washed with acetonitrile. A part (0.5 g) of the product fraction was triturated under ethanol (20 ml), filtered off and dried, yielding 0.47 g of compound (7).

### Example B.7

| | | |
|---|---|---|
| Preparation of | 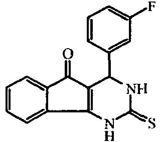 | compound (8) |

Concentrated HCl (15 drops) was added to a mixture of 3-fluorobenzaldehyde (5 mmol), indene-1,3(2H)-dione (7.5 mmol) and thiourea (5 mmol) in acetonitrile (20 ml). The resultant reaction mixture was heated in the microwave oven at 110°C for 25 minutes. A precipitate was formed, which was filtered off and washed with acetonitrile, giving 350 mg of compound (8).

### Example B.8

| | | |
|---|---|---|
| Preparation of | 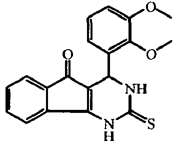 | compound (9) |

A mixture of indene-1,3(2H)-dione (5 mmol), indene-1,3(2H)-dione (7.5 mmol) and thiourea (5 mmol) in acetonitrile (20 ml) with concentrated HCl (15 drops) was heated in a microwave oven at 110°C for 22 minutes. The reaction mixture was stirred overnight at 80°C and then the precipitate was filtered off hot, washed with acetonitrile, then dried. This fraction was triturated for 2 hours at 80°C under methnol (8 ml), filtered off and dried, yielding 0.29 g of compound (9).

### Example B.9

| | | |
|---|---|---|
| Preparation of | 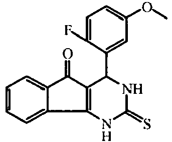 | compound (10) |

A mixture of 2-fluoro-5-methoxybenzaldehyde (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in a microwave oven at 110°C for 25 minutes. A precipitate was formed, which was filtered off and washed with acetonitrile. This fraction was triturated under methanol, filtered off and dried, yielding 0.53 g of compound (10).

### Example B.10

| | | |
|---|---|---|
| Preparation of | 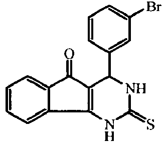 | compound (11) |

A mixture of 3-bromobenzaldehyde (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in a microwave oven at 110°C for 25 minutes. The reaction mixture was stirred at 80°C for another 2 hours and then filtered off while hot and washed with acetonitrile. The obtained fraction was triturated under methanol (10 ml) for 2 hours at 80°C. The precipitate was filtered off and dried, yielding 0.73 g of compound (11).

### Example B.11

| | | |
|---|---|---|
| Preparation of | 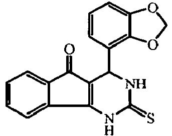 | compound (12) |

A mixture of 1,3-benzodioxole-4-carboxaldehyde (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in the microwave oven at 110°C for 22 minutes. The reaction mixture was then stirred for another 2 hours at 80°C outside of the microwave oven. A yellow orange precipitate was formed, which was filtered off and washed with acetonitrile. A part of this product fraction was treated at 80°C with IN aq. HCl (4 ml) and methanol (2 ml) for 1.5 hours. After cooling and filtration, the product fraction was triturated under methanol, filtered off and dried, yielding 0.081 g of compound (12).

### Example B.12

| | | |
|---|---|---|
| Preparation of | 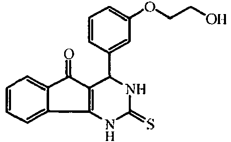 | compound (13) |

A mixture of 3-(2-hydroxyethoxy)benzaldehyde (5 mmol), indene-1,3(2H)-dione (7.5 mmol) and thiourea (7.5 mmol) in acetonitrile (20 ml) was stirred. Concentrated HCl (15 drops) was added and the reaction mixture was heated in a microwave oven at 110°C for 22 minutes. The reaction mixture was stirred for another 2 hours at 80°C. A precipitate was formed, which was filtered off hot and washed with acetonitrile. This fraction was triturated under methanol, then the precipitate was filtered off and dried, yielding 0.366 g of compound (13).

### Example B.13

| | | |
|---|---|---|
| Preparation of | 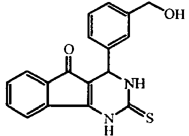 | compound (14) |

A mixture of 3-(hydroxymethyl)benzaldehyde (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in a microwave oven at 110°C for 22 minutes. The reaction mixture was then stirred another 2 hours at 80°C outside of the microwave oven. A yellow orange precipitate was formed after stirring at room temperature for another 24 hours, which was filtered off and washed with acetonitrile. This product fraction was triturated under methanol, filtered off and dried, yielding 0.200 g of compound (14).

### Example B.14

| | | |
|---|---|---|
| Preparation of | 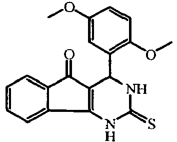 | compound (15) |

A mixture of 2,5-dimethoxybenzaldehyde (5 mmol), indene-1,3(2H)-dione (7.5 mmol) and thiourea (7.5 mmol) in acetonitrile (20 ml) was stirred at room temperature. Concentrated HCl (15 drops) was added and the reaction mixture was heated in a microwave oven at 110°C for 22 minutes. The reaction mixture was stirred for another 2 hours at 80°C. A precipitate was formed, which was filtered off hot and washed with acetonitrile. The obtained fraction was triturated under methanol, filtered off and dried, yielding 0.380 g of compound (15).

### Example B.15

| | | |
|---|---|---|
| Preparation of | 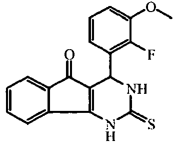 | compound (16) |

A mixture of 2-fluoro-3-methoxybenzaldehyde (5 mmol), indene-1,3(2H)-dione (7.5 mmol) and thiourea (7.5 mmol) in acetonitrile (20 ml) was stirred. Concentrated HCl (15 drops) was added and the reaction mixture was heated in a microwave oven at 110°C for 22 minutes. The reaction mixture was stirred for another 2 hours at 80°C. A precipitate was formed, which was filtered off hot and washed with acetonitrile. The fraction was triturated under methanol, filtered off and dried, yielding 0.340 g of compound (16).

### Example B.16

| | | |
|---|---|---|
| Preparation of | 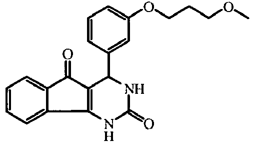 | compound (17) |

A mixture of intermediate (2) (2.3 mmol), indene-1,3(2H)-dione (3.5 mmol) and urea (2.3 mmol) in acetonitrile (15 ml) was stirred at room temperature. Concentrated HCl (7 drops) was added and the reaction mixture was heated in a microwave oven at 110°C for 30 minutes. Then the reaction mixture was stirred overnight at 80°C outside of the microwave oven. The yellow precipitate was filtered off, washed with acetonitrile and dried in vacuo, yielding 0.180 g of compound (17).

### Example B.17

| | | |
|---|---|---|
| preparation of | 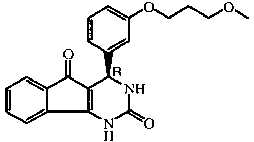 | compound (18) |
| and | 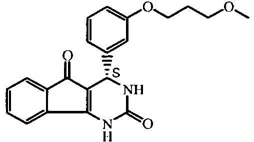 | compound (19) |

Compound (17) (0.329 mmol) was separated into enantiomers by preparative SFC purification (column: Diacel AS-H 20x250mm, mobile phase: 35% MeOH/65% CO₂ + 0.2% isopropylamine, isocratic at 40°C and 100 bar). Two product fraction groups were collected. Both fractions were evaporated and each residue was crystallized from acetonitrile, filtered off and dried under vacuum, yielding 43 mg of compound (18) and 35 mg of compound (19).

### Example B.18

| | | |
|---|---|---|
| Preparation of | 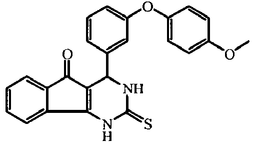 | compound (20) |

A mixture of 2,3-dihydro-1,4-benzodioxin-5-carboxaldehyde (2.5 mmol), 1,3-cyclopentanedione (3.75 mmol) and thiourea (2.5 mmol) in acetonitrile (20 ml) was stirred at room temperature. Concentrated HCl (10 drops) was added and the reaction mixture was heated in a microwave oven at 110°C for 22 minutes. The reaction mixture was stirred for another 2 hours at 80°C. A precipitate was formed, which was filtered off hot and washed with acetonitrile. This fraction was triturated under hot methanol, filtered off and dried, yielding 0.195 g of compound (20).

### Example B.19

| | | |
|---|---|---|
| Preparation of | 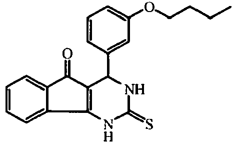 | compound (21) |

A mixture of thiourea (5 mmol), intermediate (3) (5 mmol), indene-1,3(2H)-dione (5 mmol) and HCl (6 drops) in acetonitrile (20 ml) was heated for 25 minutes to 110°C in a microwave oven, then stirred and heated overnight at 80°C out of the microwave oven. The mixture was filtered hot and the solid was treated with methanol at 80°C reflux for 2 hours. The obtained precipitate was filtered off hot, yielding 0.650 g of compound (21).

### Example B.20

| | | |
|---|---|---|
| Preparation of | 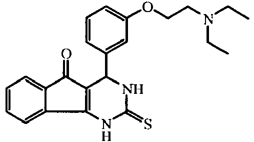 | compound (22) |

A mixture of 3-(2-diethylaminoethoxy)benzaldehyde (5 mmol), indene-1,3(2H)-dione (7.5 mmol) and thiourea (5 mmol) in acetonitrile (20 ml) was stirred at room temperature. Concentrated HCl (15 drops) was added and the reaction mixture was heated in a microwave oven at 110°C for 30 minutes. The reaction mixture was heated another 30 minutes at 110°C and was then concentrated in vacuo. The residue was purified by HPLC method B. The desired product fraction was collected separately by decantation. The obtained solid was dissolved in methanol and the solution was concentrated again, yielding 0.075 g of compound (22).

### Example B.21

| | | |
|---|---|---|
| preparation of | 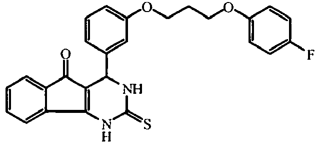 | compound (23) |

A mixture of intermediate (4) (2.5 mmol), indene-1,3(2H)-dione (3.75 mmol) and thiourea (2.5 mmol) in acetonitrile (20 ml) was stirred at room temperature. Concentrated HCl (10 drops) was added and the reaction mixture was heated in a microwave oven at 110°C for 25 minutes. The reaction mixture was stirred for another 2 hours at 80°C. A precipitate was formed, which was filtered off hot and washed with acetonitrile, filtered off and dried. This fraction was triturated under hot methanol, filtered off and dried, yielding 0.423 g of compound (23).

### Example B.22

| | | |
|---|---|---|
| Preparation of | 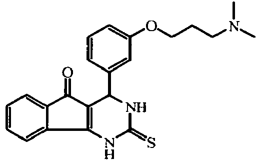 | compound (24) |

A mixture of 3-[3-(dimethylamino)propoxy]benzaldehyde (4.8 mmol), 1,3-cyclopentanedione (7.2 mmol) and thiourea (4.8 mmol) in acetonitrile (20 ml) was stirred at room temperature. Concentrated HCl (25 drops) was added and the reaction mixture was heated in a microwave oven at 110°C for 30 minutes. The reaction mixture was stirred for another 2 hours at 80°C. The reaction mixture was concentrated and purified by HPLC method B. The fractions containing product were concentrated, and the residue was triturated under methanol at 80°C, then filtered off and dried, yielding 0.050 g of compound (24).

### Example B.23

| | | |
|---|---|---|
| Preparation of | 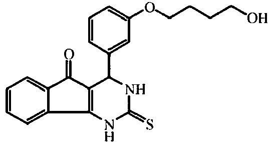 | compound (25) |
| and | 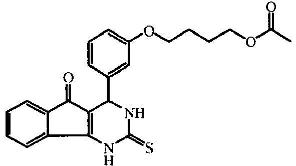 | compound (26) |

A mixture of intermediate (5) (2.5 mmol), indene-1,3(2H)-dione (3.75 mmol) and thiourea (2.5 mmol) in acetonitrile (20 ml) was stirred at room temperature. Concentrated HCl (10 drops) was added and the reaction mixture was heated in a microwave oven at 110°C for 22 minutes. The reaction mixture was stirred for another 2 hours at 80°C. A precipitate was formed, which was filtered off hot and washed with acetonitrile. This fraction was triturated under hot methanol and then purified further by HPLC method A. Two product fractions were collected and their solvent was evaporated, yielding 0.135 g of compound (25) and 0.020 g of compound (26).

### Example B.24

| | | |
|---|---|---|
| Preparation of | 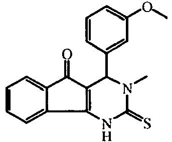 | compound (27) |

A mixture of intermediate (8) (2.19 mmol) in TFA (15 ml) was stirred in a microwave oven at 110°C for 10 min, then the reaction mixture was cooled, and the solvent was evaporated. The residue was stirred in EtOAc. The precipitate was filtered off and stirred in fresh boiling ethyl acetate, then the product fraction was filtered off and dried (vacuum, 50°C), yielding 190 mg of compound (27).

### Example B.25

| | | |
|---|---|---|
| Preparation of | 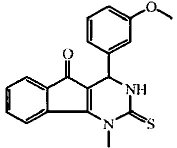 | compound (28) |

A mixture of intermediate (9) (0.219 mmol) in TFA (1.5 ml) was heated in a microwave oven for 20 minutes at 110°C. The solvent was evaporated and the residue was dissolved in ethyl acetate. The organic solution was washed with water, then the organic layer was separated, dried (MgSO₄), filtered and the solvent was evaporated. The residue was purified by HPLC method B. The product fractions were collected and the solvent was evaporated. The residue was dried under vacuum at 50°C, yielding 7 mg of compound (28).

### Example B.26

| | | |
|---|---|---|
| Preparation of | 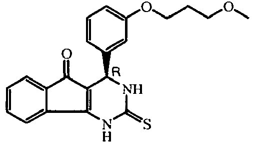 | compound (29) |
| and | 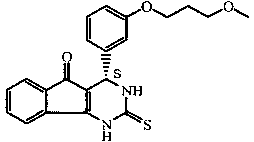 | compound (30) |

A mixture of indene-1,3(2H)-dione (7.527 mmol), 3-(3-methoxypropoxy)benzaldehyde (4.994 mmol), thiourea (0.4992 mmol) and concentrated HCl in acetonitrile (20 ml) was heated in a microwave at 110°C for 25 minutes. The resulting precipitate was filtered off and suspended in boiling EtOH. The precipitate was filtered off (while the mixture still was hot), then dried under vacuum at 50°C. The obtained fraction was separated into its enantiomers by preparative SFC purification (column: Diacel AS-H 30x250mm, mobile phase: 40% MeOH/60% CO₂ + 0.2% isopropylamine, isocratic at 40°C and 100 bar). Two product fractions were collected and their solvent was evaporated. Each residue was crystallized from acetonitrile, filtered off and dried under vacuum, yielding compound (29) and compound (30).

### Example B.27

| | | |
|---|---|---|
| Preparation of | 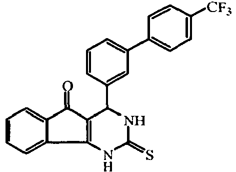 | compound (31) |

A mixture of indene-1,3(2H)-dione (6 mmol), 4'-trifluoromethylbiphenyl-3-carboxaldehyde (3.996 mmol), thiourea (3.996 mmol) and concentrated HCl in acetonitrile (20 ml) was heated in a microwave oven for 30 minutes at 110°C. The resultant solid was filtered off and dried under vacuum. This product fraction was stirred in boiling methanol, filtered off hot, then dried under vacuum at 50°C, yielding 340 mg of compound (31).

### Example B.28

| | | |
|---|---|---|
| Preparation of | 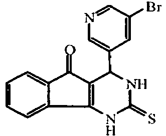 | compound (32) |

A mixture of indene-1,3(2H)-dione (5.5 mmol), 5-bromo-3-pyridinecarboxaldehyde (5 mmol), thiourea (20 mmol) and calcium chloride (0.5 mmol) in DMF (6 ml) was reacted in a microwave oven at 130°C for 80 minutes. The reaction mixture was cooled and then poured into water. The resulting precipitate was filtered off and stirred in boiling acetonitrile. The precipitate was filtered off again and dried (vacuum, 50°C). The obtained fraction was purified by high-performance liquid chromatography. The product fractions were collected and the formed precipitate was filtered off, washed with water, then dried under vacuum at 50°C. The product fraction was stirred in boiling methanol, filtered off hot, then dried under vacuum at 50°C, yielding 100 mg of compound (32).

Table 1 lists the compounds that were prepared according to one of the above Examples.

**Table 1 :**

| | |
|---|---|
| 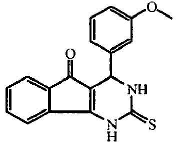 | 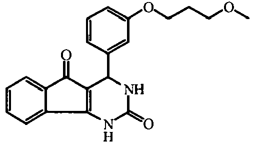 |
| Co.No.1; Ex. B.1 | Co.No.17; Ex. B.16 |
| 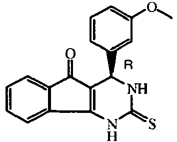 | 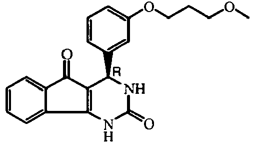 |
| Co.No.2; Ex. B.2 | Co.No.18; Ex. B.17 |
| 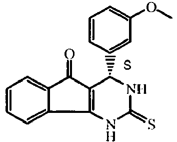 | 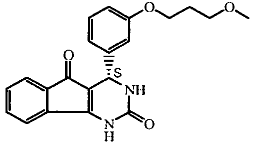 |
| Co.No.3; Ex. B.2 | Co.No.19; Ex. B.17 |
| 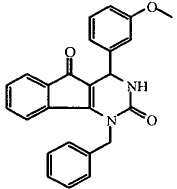 | 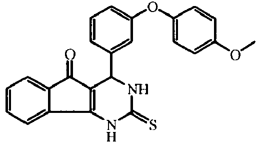 |
| Co.No.4; Ex. B.3 | Co.No.20; Ex. B.18 |
| 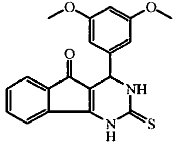 | 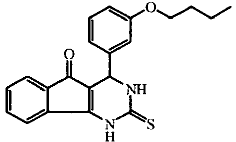 |
| Co.No.5; Ex. B.4 | Co.No.21; Ex. B.19 |
| 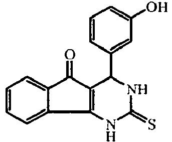 | 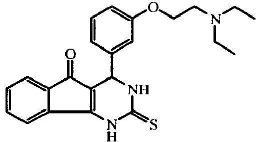 |
| Co.No.6; Ex. B.5 | Co.No.22; Ex. B.20 |
| 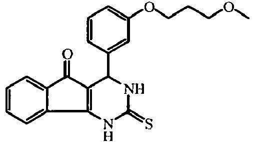 | 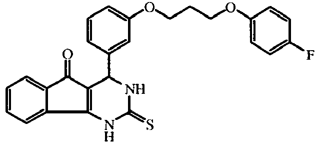 |
| Co.No.7; Ex. B.6 | Co.No.23; Ex. B.21 |
| 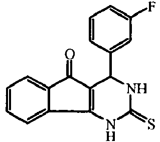 | 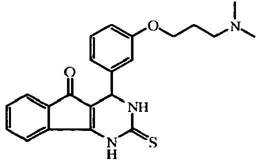 |
| Co.No.8; Ex. B.7 | Co.No.24; Ex. B.22 |
| 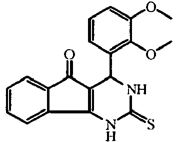 | 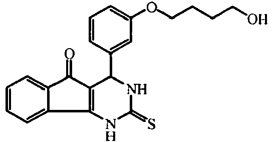 |
| Co.No.9; Ex. B.8 | Co.No.25; Ex. B.23 |
| 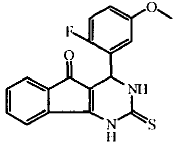 | 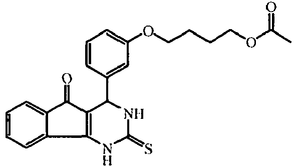 |
| Co.No.10; Ex. B.9 | Co.No.26; Ex. B.23 |
| 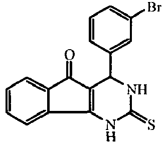 | 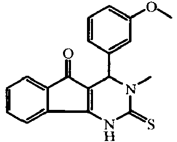 |
| Co.No.11; Ex. B.10 | Co.No.27; Ex. B.24 |
| 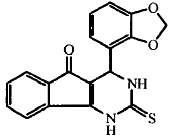 | 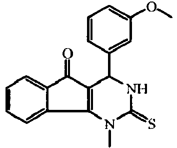 |
| Co.No.12; Ex. B.11 | Co.No.28; Ex. B.25 |
| 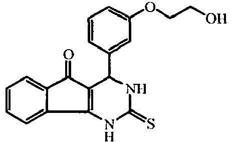 | 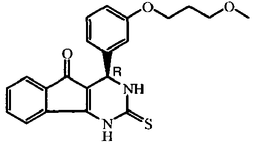 |
| Co.No.13; Ex. B.12 | Co.No.29; Ex. B.26 |
| 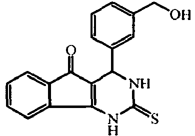 | 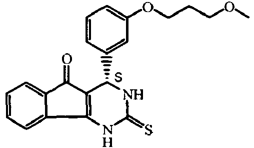 |
| Co.No.14; Ex. B.13 | Co.No.30; Ex. B.26 |
| 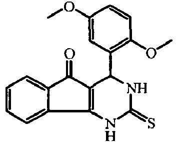 | 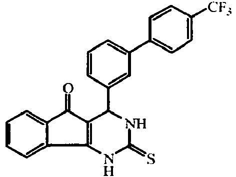 |
| Co.No.15; Ex. B.14 | Co.No.31; Ex. B.27 |
| 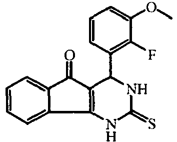 | 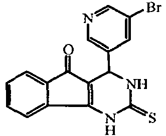 |
| Co.No.16; Ex. B.15 | Co.No.32; Ex. B.28 |

### HPLC Method A

The product was purified by reversed phase high-performance liquid chromatography (Shandon Hyperprep^{®} C18 BDS (Base Deactivated Silica) 8 µm, 250 g, I.D. 5 cm). Three mobile phases were used (phase A: 90 % of a 0.5 % NH₄OAc solution in water+ 10 % CH₃CN; phase B: CH₃OH; phase C: CH₃CN). First, 75 % A and 25 % B with a flow rate of 40 ml/min was hold for 0.5 minutes. Then a gradient was applied to 50 % B and 50 % C in 41 minutes with a flow rate of 80 ml/min. Then a gradient was applied to 100 % C in 20 minutes with a flow rate of 80 ml/min and hold for 4 minutes.

### HPLC Method B

The product was purified by reversed-phase high-performance liquid chromatography (Shandon Hyperprep^{®} C18 BDS (Base Deactivated Silica) 8 µm, 250 g, I.D. 5 cm). Three mobile phases were used (phase A: a 0.25 % NH₄HCO₃ solution in water; phase B: CH₃OH; phase C: CH₃CN). First, 75 % A and 25 % B with a flow rate of 40 ml/min was hold for 0.5 minutes. Then a gradient was applied to 50 % B and 50 % C in 41 minutes with a flow rate of 80 ml/min. Then a gradient was applied to 100 % C in 20 minutes with a flow rate of 80 ml/min and hold for 4 minutes.

### HPLC Method C

The product was purified by reversed-phase high-performance liquid chromatography (Shandon Hyperprep^{®} C18 BDS (Base Deactivated Silica) 8 µm, 250 g, I.D. 5 cm). Three mobile phases were used (phase A: a 0.25 % NH₄HCO₃ solution in water; phase B: CH₃OH; phase C: CH₃CN). First, 75 % A and 25 % B with a flow rate of 40 ml/min was hold for 0.5 minutes. Then a gradient was applied to 100 % B in 41 minutes with a flow rate of 80 ml/min. Then a gradient was applied to 100 % C in 20 minutes with a flow rate of 80 ml/min and hold for 4 minutes.

### C. Analytical Part

### C.1. LC-MS General procedure 1

The LC measurement was performed using an Acquity UPLC (Waters) system comprising a binary pump, a sample organizer, a column heater (set at 55 °C), a diode-array detector (DAD) and a column as specified in the respective methods below. Flow from the column was split to a MS spectrometer. The MS detector was configured with an electrospray ionization source. Mass spectra were acquired by scanning from 100 to 1000 in 0.18 seconds using a dwell time of 0.02 seconds. The capillary needle voltage was 3.5 kV and the source temperature was maintained at 140 °C. Nitrogen was used as the nebulizer gas. Data acquisition was performed with a Waters-Micromass MassLynx-Openlynx data system.

Reversed phase UPLC (Ultra Performance Liquid Chromatography) was carried out on a bridged ethylsiloxane/silica hybrid (BEH) C18 column (1.7 µm, 2.1 x 50 mm; Waters Acquity) with a flow rate of 0.8 ml/min. Two mobile phases (mobile phase A: 0.1 % formic acid in H₂O/methanol 95/5; mobile phase B: methanol) were used to run a gradient condition from 95 % A and 5 % B to 5 % A and 95 % B in 1.3 minutes and hold for 0.2 minutes. An injection volume of 0.5 µl was used. Cone voltage was 10 V for positive ionization mode and 20 V for negative ionization mode.

**Table 2 : LCMS data - Retention time (Rₜ in minutes), (MH)⁺ peak (of the free base)**

| **Co. No.** | **Rt** | **MH+** |
|---|---|---|
| 8 | 1.14 | 311 |
| 9 | 1.11 | 353 |
| 11 | 1.23 | 371 |
| 21 | 1.33 | 365 |

### C.3 Melting Points

For a number of compounds, melting points (m.p.) were determined with a DSC823e (Mettler-Toledo). Melting points were measured with a temperature gradient of 30°C/minute. The reported values are peak values. Maximum temperature was 400 °C. Values are obtained with experimental uncertainties that are commonly associated with this analytical method.

**Table 3 : melting point**

| Co. No. | m.p. | Co. No. | m.p. |
|---|---|---|---|
| 1 | 295.22 °C | 18 | 206.47 °C |
| 2 | 252.73 °C | 19 | 207.44 °C |
| 3 | 253.60 °C | 20 | 269.04 °C |
| 4 | 188.65 °C | 22 | 243.58 °C |
| 5 | 287.08 °C | 23 | 245.01 °C |
| 6 | 288.04 °C | 24 | 237.90 °C |
| 7 | 235.68 °C | 25 | 224.49 °C |
| 10 | 289.27 °C | 26 | 199.50 °C |
| 12 | 289.34 °C | 27 | 286.94 °C |
| 13 | 261.08 °C | 29 | 172.85 °C |
| 14 | 265.63 °C | 30 | 171.86 °C |
| 15 | 295.33 °C | 31 | 149.46 °C |
| 16 | 287.05 °C | 32 | 294.61 °C |
| 17 | 251.12°C | | |

### C.4 Optical Rotation

The optical rotation was measured using a Perkin Elmer 341 polarimeter. [α]_{D}²⁰ indicates the optical rotation measured with light at the wavelength of the D-line of sodium (589 nm) at a temperature of 20°C^{*}. The cell pathlength is 10 cm. Behind the actual value the concentration and solvent of the solution which was used to measure the optical rotation are mentioned.

**Table 4 : optical rotation**

| **Co. No.** | **Specific optical rotation [α]_{D}²⁰** |
|---|---|
| 2 | +605.19 ° (589 nm, c 0.2158 w/v %, DMF, 20 °C) |
| 3 | -623.67 ° (589 nm, c 0.2264 w/v %, DMF, 20 °C) |
| 18 | +349.06 ° (589 nm, c 0.1166 w/v %, MeOH, 20 °C) |
| 19 | -363.73 ° (589 nm, c 0.0932 w/v %, MeOH, 20 °C) |
| 29 | +588.75 ° (578 nm, c 0.1938 w/v %, DMF, 20 °C) |
| 30 | -581.71 ° (578 nm, c 0.2876 w/v %, DMF, 20 °C) |

### D. Pharmacological examples

### Cell and Culture

The Human TRPA1 gene was cloned into the pT-REx-Dest30 inducible vector and afterwards stably transfected in T-Rex™-293 cells (purchased from Invitrogen, Merelbeke, Belgium). This tetracyclin inducible hTRPA1 expression system was used in order to prevent Ca²⁺ overload in the cultured cells due to sustained TRPA1 expression. hTRPA1/TREx-HEK293 cells (referred to as hTRPA1 cells in the following text) were maintained under standard sterile cell culture conditions. The culture medium for the hTRPA1-HEK cells was DMEM (Gibco BRL, Invitrogen, Merelbeke, Belgium) supplemented with 0.5 g/l geneticin (Gibco), 5 mg/l blasticidin (Invitrogen), 14.6 g/l L-Glutamine (200 mM; Gibco), 5 g/l penicillin/streptomycin (5.10⁻⁶ IU/l, Gibco), 5.5 g/l pyruvic acid (Gibco) and 10% foetal calf serum (Hyclone, Logan UT, USA).

### Ca2+ fluorometry

Binding of an agonist to the TRPA1 ion-channel activates and opens the ion-channel which causes a robust increase in intracellular Ca²⁺ concentration. For detecting and measuring intracellular Ca²⁺ concentration, cells were loaded with a Ca²⁺-sensitive dye. Changes in fluorescence in the cell, that correspond to changes in Ca²⁺ concentration in the cell, can kinetically be monitored with the FDSS instrument (Hamamatsu) and are indicative for agonism towards the TRPA1 ion channel and this can be reduced by TRPA1- receptor antagonists.

For the fluorometric Ca²⁺ measurements hTRPA1-HEK cells were resuspended in HBSS seeding medium: HBSS (with CaCl₂ and MgCl₂; Gibco) supplemented with 14.6 g/l L-Glutamine (200 mM; Gibco), 5 g/l penicillin/streptomycin (5.10⁻⁶ IU/l, Gibco), 5.5 g/l pyruvic acid (Gibco), 5 mM HEPES (Gibco), 5 ml Insulin-Transferrin-Xelenium-x (Gibco) and 10% foetal calf serum (heat inactivated for 30 minutes at 56°C; Hyclone, Logan UT, USA). The cells were seeded in poly-D-lysine-coated 384-well round bottom polypropylene plates (Costar Coming, Data Packaging, Cambridge MA, USA) at 12000 cells/well. 50 ng/ml tetracycline was added to induce the hTRPA1 expression 24 h before the experiment.

The cells were loaded with 5mg/l Fluo4-AM (Molecular Probes, Invitrogen, Merelbeke, Belgium) dissolved in HBSS seeding medium supplemented with 0.7 g/l Probenecid (Sigma) and incubated for 1 h at 37°C and subsequently at 20 °C for 1 to 2 h. The fluorescence was measured in the FDSS 6000 imaging based plate reader (Hamamutsu Photonics K.K., Hamamutsu City, Japan). The excitation wavelength was 488 nm and the emission wavelength 540 nm. After a control period of 12 seconds the JNJ compounds were added and the Ca²⁺ signal was measured within 14 minutes after application. Finally, the control TRPA1 agonist 11H-dibenzo[b,e]azepine-10-carboxylic acid methyl ester [the synthesis of which is hereafter described as compound (D-6)] or BITC (benzylisothiocyanate, FLUKA) was added at a final concentration of 25 nM. This protocol allowed to study the agonism (in the time window where TRPA1 agonist compounds were added) and the antagonism (by checking whether the pre-application of the compounds reduced the agonistic affect of the control agonist). The emission ratio was calculated by dividing the emission signal (Em₅₄₀) by the first Em₅₄₀ signal of the control period to compensate for the background fluorescence. On every 384 well plate 4 series of DMSO control experiments were performed of which 2 with and 2 without control agonist 11H-dibenzo[b,e]azepine-10-carboxylic acid methyl ester or BITC. For intracellular Ca²⁺ measurements compound stock solutions (10 or 100 mM) were further diluted in DMSO in order to obtain finally 1 % DMSO in the extracellular solution.

Antagonism values of the described compounds determined with 11H-dibenzo[b,e]-azepine-10-carboxylic acid methyl ester or BITC were found to be in good agreement with each other. The IC50 values measured using 11H-dibenzo[b,e]azepine-10-carboxylic acid methyl ester as TRPA1 agonist are listed in Table 5.

**Table 5 : IC50 values for TRPA1 antagonism**

| Co. No. | IC50 hTRPA1 | Co. No. | IC50 hTRPA1 |
|---|---|---|---|
| 1 | ~128 nM | 17 | ~1688 nM |
| 2 | ~76 nM | 18 | 1318 nM |
| 3 | >10 µM | 19 | >10 µM |
| 4 | ~6026 nM | 20 | 1462 nM |
| 5 | ~2775 nM | 21 | 314 nM |
| 6 | ~636 nM | 22 | 1202 nM |
| 7 | 47 nM | 23 | 4860 nM |
| 8 | ~1063 nM | 24 | >10 µM |
| 9 | >10 µM | 25 | ~685 nM |
| 10 | ~365 nM | 26 | 557 nM |
| 11 | 495 nM | 27 | >10 µM |
| 12 | ~4406 nM | 28 | ~85 nM |
| 13 | 213 nM | 29 | ~13 nM |
| 14 | ~708 nM | 30 | >10 µM |
| 15 | >10 µM | 31 | >10 µM |
| 16 | ~287 nM | 32 | 1669 nM |

### Synthesis of the TRPA1 agonist 11H-dibenzo[b,e]azepine-10-carboxylic acid methyl ester (compound (D-6) - reference example

| | | |
|---|---|---|
| a) Preparation of | 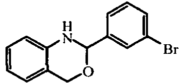 | compound (D-1) |

A mixture of 2-amino-benzenemethanol (0.073 mol) and 3-bromo-benzaldehyde (0.073 mol) in 2-propanol (100 mL) was stirred for 3 hours at room temperature. The solvent was evaporated. Part (3 g) of the residue (20.5 g) was crystallized from hexane. The precipitate was filtered off and dried, yielding 1.37 g of compound (D-1).

| | | |
|---|---|---|
| b) Preparation of | 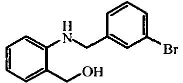 | compound (D-2) |

Reaction under nitrogen atmosphere. Sodium borohydride (0.1172 mol) was added slowly to a mixture of compound (D-1) (0.0586 mol) in ethanol (200 mL). The reaction mixture was stirred and refluxed for 1 hour. The mixture was cooled on an ice-water bath, quenched with NH₄Cl 20% and extracted with CH₂Cl₂. The organic layer was dried, filtered and the solvent was evaporated, yielding 14.8 g of compound (D-2).

| | | |
|---|---|---|
| c) Preparation of | 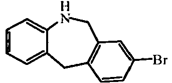 | compound (D-3) |
| and | 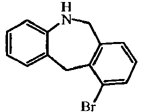 | compound (D-4) |

A solution of compound (D-2) (0.180 mol) in CH₂Cl₂ (50 mL) was added over a one-hour period to a cooled (± -10 to -20°C) solution of concentrated H₂SO₄ (500 mL). Then the ice-bath was removed, and the mixture was stirred for one hour at room temperature. The reaction mixture was added to ice-water, cooled on ice, and alkalized with a 50% aqueous NaOH solution. The resulting mixture (± 3 L) was extracted with CH₂Cl₂. The organic layer was separated, dried on MgSO₄, filtered and the filtrate was concentrated in vacuo. A part (8 g) of this residue was purified via Supercritical Fluid Chromatography (SFC, column: Diacel AD-H 30 x 250mm, mobile phase: 55% MeOH/45% CO₂ + 0.2% isopropylamine, 40°C, 100 bar) to give 2 g of compound (D-4) (7-bromo-isomer) and 4.65 g of compound (D-3) (9-bromo-isomer).

| | | |
|---|---|---|
| d) Preparation of | 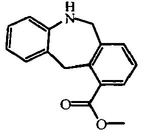 | compound (D-5) |

A mixture of compound (D-4) (0.008 mol), potassium acetate (4 g), Pd(OAc)₂ (0.04 g) and 1,1'-(1,3-propanediyl)bis[1,1-diphenyl- phosphine (0.16 g) in methanol (100 mL) and THF (100 mL) was placed in a pressure reactor and pressurized with CO gas up to 50 kg/square cm. The reaction mixture was heated at 125°C for 16 hours, then cooled, filtered over dicalite, and the solvent was evaporated. The residue was partitioned between CH₂Cl₂ and water. The organic layer was dried over MgSO₄, filtered, then the filtrate was concentrated. The residue was purified by column chromatography over silica gel (eluent: CH₂Cl₂). The desired fractions were collected and the solvent was evaporated, yielding 1.86 g of compound (D-5).

| | | |
|---|---|---|
| e) Preparation of | 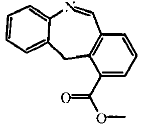 | compound (D-6) |

A mixture of compound (D-5) (0.00735 mol) and manganese oxide (0.0367 mol) in toluene (20 mL) was stirred at 90°C for 4 hours. The reaction mixture was filtered over a silica gel pad (eluent: toluene, then CH₂Cl₂). The (yellow) CH₂Cl₂ layer was concentrated. The residue was purified by column chromatography over silica gel (eluent: heptane/EtOAc 100/0 to 70/30). The product fractions were collected and the solvent was evaporated. The residue was triturated under DIPE, filtered off and dried, yielding 1.35 g of 11H-dibenzo[b,e]azepine-10-carboxylic acid methyl ester as compound (D-6).

## Claims

1. Compound of formula (I) 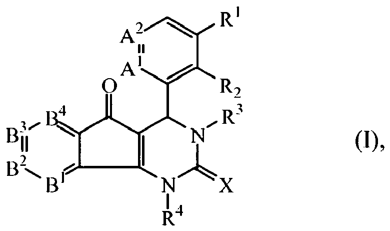 including any stereochemically isomeric form thereof wherein
A¹ and A² are both CR⁵, or one of A¹ or A² is N and the other is CR⁵, wherein each R⁵ is independently selected from hydrogen, halo, C₁₋₆alkyl, C₁₋₆alkyloxy, polyhaloC₁₋₆alkyl or polyhaloC₁₋₆alkyloxy;
B¹, B² B³ and B⁴ are all CH, or one of B¹, B² B³ and B⁴ is N and the other are CH;
X is O or S;
R¹ is halo, hydroxy, cyano, amino, C₁₋₆alkyl, hydroxyC₁₋₆alkyl, C₁₋₆alkyloxy, polyhaloC₁₋₆alkyloxy, aryl, aryloxy,
or OR⁶ wherein R⁶ is C₁₋₆alkyl substituted with hydroxy, C₁₋₄alkyloxy, aryl, aryloxy, C₁₋₄alkylcarbonyl, C₁₋₄alkylcarbonyloxy, or NR⁷R⁸ wherein R⁷ and R⁸ are each independently selected from hydrogen or C₁₋₄alkyl;
R² is hydrogen, fluoro, C₁₋₄alkyl or C₁₋₄alkyloxy;
and R¹ and R² may be taken together to form a -O-CH₂-O- or -O-CH₂-CH₂-O-radical;
R³ is hydrogen, C₁₋₄alkyl, or arylC₁₋₂alkyl;
R⁴ is hydrogen, C₁₋₄alkyl, or arylC₁₋₂alkyl;
each aryl is independently from the other selected from a phenyl substituted with 1, 2 or 3 substituents each independently selected from hydrogen, halo, hydroxy, C₁₋₄alkyl, polyhaloC₁₋₄alkyl, C₁₋₄alkyloxy, polyhaloC₁₋₄alkyloxy, cyano, nitro, amino, or mono- or di-(C₁₋₄alkyl)amino;
provided that when X is O and A¹ and A² are both CR⁵ wherein R⁵ is hydrogen and R² is hydrogen or C₁₋₄alkyloxy, and R³ and R⁴ are hydrogen, than R¹ is not halo, C₁₋₆alkyl or C₁₋₆alkyloxy;
or a pharmaceutically acceptable acid addition salt thereof, or a solvate thereof, or an N-oxide thereof.

2. The compound as claimed in claim 1 wherein B¹, B², B³ and B⁴ are all CH.

3. The compound as claimed in claim 1 wherein X is S.

4. The compound as claimed in claim 1 wherein A¹ and A² are both CR⁵ wherein each R⁵ is independently selected from hydrogen or halo.

5. The compound as claimed in claim 1 wherein the compound has the R-configuration at the chiral carbon atom marked with a *

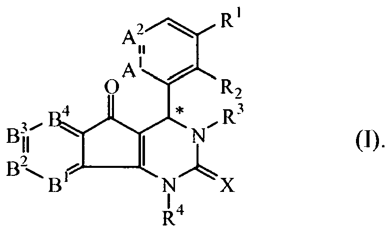

6. A pharmaceutical composition comprising at least one pharmaceutically acceptable carrier and a therapeutically active amount of a compound according to any of claims I to 5.

7. A process for preparing a pharmaceutical composition according to claim 6 wherein a therapeutically active amount of a compound according to any of claims 1 to 5 is intimately mixed with a pharmaceutically acceptable carrier.

8. A compound according to any of claims 1 to 5 for use as a medicine.

9. A process for preparing a compound of formula (I) which can be prepared
a) by a condensation reaction between a 1,3-dicarbonyl intermediate (III), an aldehyde (IV), and (thio)urea (V), 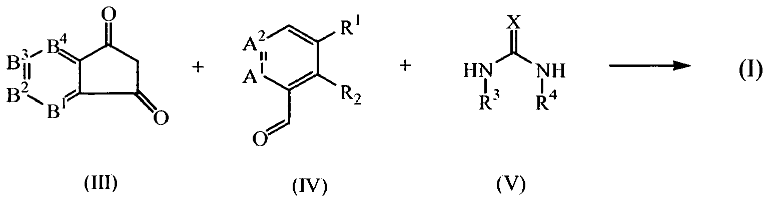
or b) compounds of formula (I) are converted into each other following art-known transformation reactions; or if desired; a compound of formula (I) is converted into a pharmaceutically acceptable acid addition salt, or conversely, an acid addition salt of a compound of formula (I) is converted into a free base form with alkali; and, if desired, preparing stereochemically isomeric forms thereof.

## Patentansprüche

1. Verbindung der Formel (I) 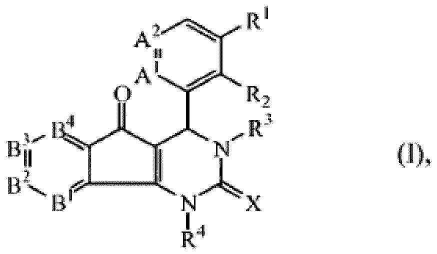 einschließlich beliebiger stereochemisch isomerer Formen davon, wobei
A¹ und A² beide für CR⁵ stehen oder eine der Variablen A¹ und A² für N steht und die andere für CR⁵ steht, wobei R⁵ jeweils unabhängig aus Wasserstoff, Halogen, C₁₋₆-Alkyl, C₁₋₆-Alkyloxy, Polyhalogen-C₁₋₆-alkyl oder Polyhalogen-C₁₋₆-alkyl-oxy ausgewählt ist;
B¹, B², B³ und B⁴ alle für CH stehen oder eine der Variablen B¹, B², B³ und B⁴ für N steht und die anderen für CH stehen;
X für O oder S steht;
R¹ für Halogen, Hydroxy, Cyano, Amino, C₁₋₆-Alkyl, Hydroxy-C₁₋₆-alkyl, C₁₋₆-Alkyloxy, Polyhalogen-C₁₋₆-alkyloxy, Aryl, Aryloxy oder OR⁶ steht, wobei R⁶ für C₁₋₆-Alkyl steht, das durch Hydroxy, C₁₋₄-Alkyloxy, Aryl, Aryloxy, C₁₋₄-Alkylcarbonyl, C₁₋₄-Alkylcarbonyloxy oder NR⁷R⁸ substituiert ist, wobei R⁷ und R⁸ jeweils unabhängig aus Wasserstoff oder C₁₋₄-Alkyl ausgewählt sind;
R² für Wasserstoff, Fluor, C₁₋₄-Alkyl oder C₁₋₄-Alkyloxy steht;
und R¹ und R² zusammengenommen einen -O-CH₂O- oder -O-CH₂-CH₂-O-Rest bilden können;
R³ für Wasserstoff, C₁₋₄-Alkyl oder Aryl-C₁₋₂-alkyl steht;
R⁴ für Wasserstoff, C₁₋₄-Alkyl oder Aryl-C₁₋₂-alkyl steht;
Aryl jeweils unabhängig voneinander aus einem Phenyl ausgewählt ist, das durch 1, 2 oder 3 Substituenten substituiert ist, die jeweils unabhängig aus Wasserstoff, Halogen, Hydroxy, C₁₋₄-Alkyl, Polyhalogen-C₁₋₄-alkyl, C₁₋₄-Alkyloxy, Poly-halogen-C₁₋₄-alkyloxy, Cyano, Nitro, Amino oder Mono- oder Di(C₁₋₄-alkyl)amino ausgewählt sind;
mit der Maßgabe, dass dann, wenn X für O steht und A¹ und A² beide für CR⁵ stehen, wobei R⁵ für Wasserstoff steht, und R² für Wasserstoff oder C₁₋₄-Alkyloxy steht und R³ und R⁴ für Wasserstoff stehen, R¹ nicht für Halogen, C₁₋₆-Alkyl oder C₁₋₆-Alkyloxy steht;
oder ein pharmazeutisch unbedenkliches Säureadditionssalz davon oder ein Solvat davon oder ein N-Oxid davon.

2. Verbindung nach Anspruch 1, wobei B¹, B², B³ und B⁴ alle für CH stehen.

3. Verbindung nach Anspruch 1, wobei X für S steht.

4. Verbindung nach Anspruch 1, wobei A¹ und A² beide für CR⁵ stehen, wobei R⁵ jeweils unabhängig aus Wasserstoff oder Halogen ausgewählt ist.

5. Verbindung nach Anspruch 1, wobei die Verbindung an dem mit einem * markierten chiralen Kohlenstoffatom die R-Konfiguration aufweist

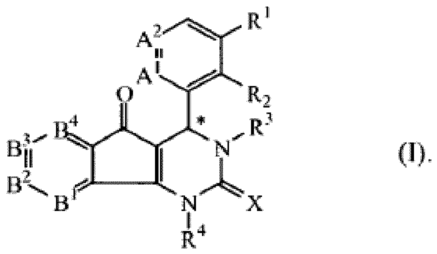

6. Pharmazeutische Zusammensetzung, umfassend mindestens einen pharmazeutisch unbedenklichen Träger und eine therapeutisch wirksame Menge einer Verbindung nach einem der Ansprüche 1 bis 5.

7. Verfahren zur Herstellung einer pharmazeutischen Zusammensetzung nach Anspruch 6, bei dem man eine therapeutisch wirksame Menge einer Verbindung nach einem der Ansprüche 1 bis 5 innig mit einem pharmazeutisch unbedenklichen Träger mischt.

8. Verbindung nach einem der Ansprüche 1 bis 5 zur Verwendung als Medizin.

9. Verfahren zur Herstellung einer Verbindung der Formel (I), die hergestellt werden kann a) durch eine Kondensationsreaktion zwischen einem 1,3-Dicarbonyl-Zwischenprodukt (III), einem Aldehyd (IV) und (Thio)harnstofff (V), 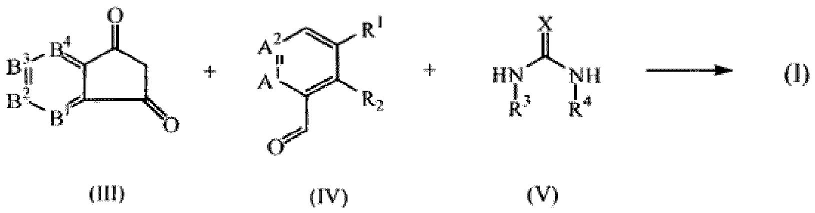 oder b) Verbindungen der Formel (I) werden nach an sich bekannten Transformationsreaktionen ineinander umgewandelt; oder gegebenenfalls wird eine Verbindung der Formel (I) in ein pharmazeutisch unbedenkliches Säureadditionssalz umgewandelt oder umgekehrt ein Säureadditionssalz einer Verbindung der Formel (I) mit Alkali in eine freie Basenform umgewandelt; und gegebenenfalls werden stereochemisch isomere Formen davon hergestellt.

## Revendications

1. Composé de formule (I) 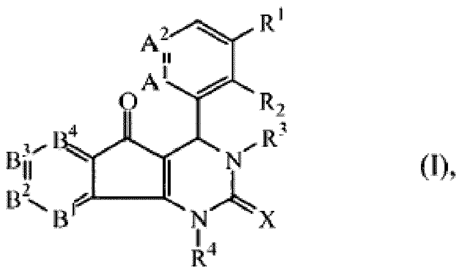 y compris toute forme stéréochimiquement isomère de celui-ci, dans laquelle
A¹ et A² sont tous deux CR⁵, ou l'un de A¹ ou A² est N et l'autre est CR⁵, où
chaque R⁵ est choisi indépendamment parmi hydrogène, halogéno, C₁₋₆alkyle, C₁₋₆alkyloxy, polyhalogénoC₁₋₆alkyle ou polyhalogénoC₁₋₆alkyloxy ;
B¹, B², B³ et B⁴ sont tous CH, ou l'un de B¹, B², B³ et B⁴ est N et les autres sont CH ;
X est O ou S ;
R¹ est halogéno, hydroxy, cyano, amino, C₁₋₆alkyle, hydroxyC₁₋₆alkyle, C₁₋₆alkyloxy, polyhalogénoC₁₋₆alkyloxy, aryle, aryloxy,
ou OR⁶, où R⁶ est C₁₋₆alkyle substitué par hydroxy, C₁₋₄alkyloxy, aryle, aryloxy, C₁₋₄alkylcarbonyle, C₁₋₄alkylcarbonyloxy, ou NR⁷R⁸ où R⁷ et R⁸ sont choisis chacun indépendamment parmi hydrogène ou C₁₋₄alkyle ;
R² est hydrogène, fluoro, C₁₋₄alkyle ou C₁₋₄alkyloxy ;
et R¹ et R² peuvent être pris ensemble pour former un radical -O-CH₂-O- ou -O-CH₂-CH₂-O- ;
R³ est hydrogène, C₁₋₄alkyle, ou arylC₁₋₂alkyle ;
R⁴ est hydrogène, C₁₋₄alkyle, ou arylC₁₋₂alkyle ;
chaque aryle est, indépendamment l'un de l'autre, choisi parmi un phényle substitué par 1, 2 ou 3 substituants choisis chacun indépendamment parmi hydrogène, halogéno, hydroxy, C₁₋₄alkyle, polyhalogénoC₁₋₄alkyle, C₁₋₄alkyloxy, polyhalogénoC₁₋₄alkyloxy, cyano, nitro, amino, ou mono- ou di-(C₁₋₄alkyl)amino ;
à condition que lorsque X est O et A¹ et A² sont tous deux CR⁵ où R⁵ est hydrogène et R² est hydrogène ou C₁₋₄alkyloxy, et R³ et R⁴ sont hydrogène, alors R¹ ne soit pas halogéno, C₁₋₆alkyle ou C₁₋₆alkyloxy ;
ou un sel d'addition d'acide pharmaceutiquement acceptable de celui-ci, ou un solvate de celui-ci, ou un N-oxyde de celui-ci.

2. Composé selon la revendication 1, **caractérisé en ce que** B¹, B², B³ et B⁴ sont tous CH.

3. Composé selon la revendication 1, **caractérisé en ce que** X est S.

4. Composé selon la revendication 1, **caractérisé en ce que** A¹ et A² sont tous deux CR⁵ où chaque R⁵ est choisi indépendamment parmi hydrogène ou halogéno.

5. Composé selon la revendication 1, **caractérisé en ce que** le composé a la configuration R au niveau de l'atome de carbone chirale marqué par un *

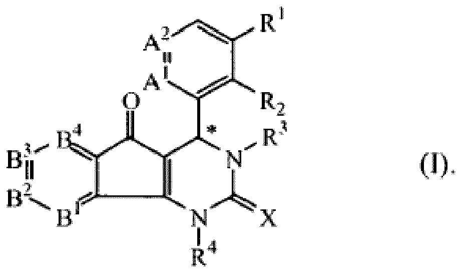

6. Composition pharmaceutique comprenant au moins un support pharmaceutiquement acceptable et une quantité thérapeutiquement active d'un composé selon l'une quelconque des revendications 1 à 5.

7. Procédé de préparation d'une composition pharmaceutique selon la revendication 6, **caractérisé en ce qu'**une quantité thérapeutiquement active d'un composé selon l'une quelconque des revendications 1 à 5 est mélangée intimement avec un support pharmaceutiquement acceptable.

8. Composé selon l'une quelconque des revendications 1 à 5, destiné à être utilisé comme médicament.

9. Procédé de préparation d'un composé de formule (I), qui peut être préparé
a) par une réaction de condensation entre un intermédiaire 1,3-dicarbonyle (III), un aldéhyde (IV), et une (thio)urée (V), 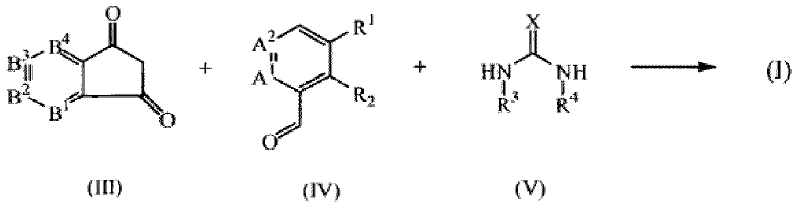 ou
b) des composés de formule (I) sont transformés les uns en les autres en suivant des réactions de transformation connues dans l'art ; ou si on le souhaite, un composé de formule (I) est transformé en un sel d'addition d'acide pharmaceutiquement acceptable, ou inversement un sel d'addition d'acide d'un composé de formule (I) est transformé en une forme base libre avec un alcali ; et, si on le souhaite, on prépare des formes stéréoisomères de celui-ci.
